# EUROPEAN PATENT APPLICATION

(11) **EP 1 652 514 A1**
(43) Date of publication of application: **03.05.2006**
(21) Application number: 04771500.8
(22) Date of filing: 04.08.2004
(51) Int. Cl.: A61K 9/16, A61K 31/4184, A61K 47/04, A61K 47/16, A61K 47/36, A61K 47/38, A61K 47/46, A61K 31/4439, A61K 45/00, A61P 1/04

(54) **AT-USE DISPERSED PREPARATION**

(30) Priority: 04.08.2003 JP 2003286229
(71) Applicant: Eisai Co., Ltd., Tokyo 112-088 (JP)
(72) Inventor: UKAI, Koji c/o Kawashima Industrial Park Eisai Co., Kakamigahara-shi, Gifu (JP); ASAI, Yasuyuki c/o Kawashima Industrial Park, Kakamigahara-shi Gifu (JP); KATO, Yoshiteru c/o Kawashima Industrial Park, Kakamigahara-shi Gifu (JP); OHWAKI, Takayuki c/o Kawashima Industrial Park, Kakamigahara-shi Gifu (JP); AOKI, Shigeru c/o Kawashima Industrial Park, Kakamigahara-shi Gifu (JP); ZAIMA, Yasuhi c/o Kawashima Industrial Park, Kakamigahara-shi Gifu (JP); YAMAGUCHI, Takehiro c/oKawashima Industrial Park, Kakamigahara-shi Gifu (JP)
(74) Representative: HOFFMANN EITLE
(86) International application number: PCT/JP2004/011515
(87) International publication number: WO 2005/011637

(57) **Abstract**

The present invention provides a pharmaceutical preparation to be dispersed before administration which has an adequate viscosity and a suitable flowability even when dispersed in a small amount of water and can be easily administered through an NG tube is provided. Specifically, the pharmaceutical preparation to be dispersed before administration contains active granules containing a pharmaceutically active substance having an average particle diameter of 5 mm or less and a thickening agent, and can be administered through an NG tube by dispersing in water before administration.

## Description

### Technical Field

The present invention relates to a pharmaceutical preparation to be dispersed before administration which has an adequate viscosity and suitable flowability even when dispersed in a small amount of water to be easily administered through an NG tube.

### Background Art

Coated tablets, coated granules or capsules containing coated granules are generally used for providing enteric abilities, masking a bitter taste and/or imparting safety to a drug in pharmaceutical preparations.

Liquid preparations, water-soluble granules and powders are widely used as regular pediatric pharmaceutical preparations. (1) These pharmaceutical preparations fail to impart enteric abilities, bitterness masking or safety to a drug as described above. In addition, (2) it is difficult for children to take tablets or capsules. Accordingly, coated granules are widely used. These coated granules are taken with water. Children of some age or elder (for example, of two years old or elder) can take such coated granules by taking coated granules previously in mouth and then drink water, or by dispersing the coated granules in water and drinking the dispersion as intact, as in adults.

### Disclosure of Invention

As for infants, however, it is difficult to let them take coated granules previously in mouth and then drink water. When coated granules are dissolved or dispersed in water before administration and then taken, the following problems occur.
(1) Coated granules are difficult to administer, since granules sink down at the bottom of the dispersion.
(2) When the coated granules are dispersed in a small amount of water, the resulting dispersion has low flowability and is difficult to take.
(3) If the coated granules are dispersed in a large amount of water, the dispersion has sufficient flowability. Such a large amount of water, however, cannot be used because infants have a small capacity of the stomach.
(4) When a dispersion containing the coated granules in water is administered to babies by the age of about one month through an NG tube (nasogastric tube) passing through the nose, it is difficult to administer, because the dispersion as intact has an excessively low viscosity.

A demand has therefore been made on a pharmaceutical preparation to be dispersed before administration which has an adequate viscosity and suitable flowability even when dispersed in a small amount of water and can thereby be easily administered through an NG tube.

After intensive investigations to solve these problems, the present inventors has achieved the present invention. Accordingly, an object of the present invention is to provide a pharmaceutical preparation to be dispersed before administration which has an adequate viscosity and suitable flowability even when dispersed in a small amount of water and can be easily administered through an NG tube.

Specifically, the present invention provides a pharmaceutical preparation to be dispersed before administration, containing a active granules including a pharmaceutically active substance and having an average particle diameter of 2 mm or less, and a thickening agent, wherein the pharmaceutical preparation is capable of being administered through an NG tube by dispersing in water before administration.

The present invention relates to a method of administering a pharmaceutical preparation to be dispersed before administration, containing a active granules including a pharmaceutically active substance and having an average particle diameter of 2 mm or less, and a thickening agent, which method comprises dispersing the pharmaceutical preparation in water before administration; and administering through an NG tube.

### Detailed Description of the Invention

The "pharmaceutical preparation to be dispersed before administration" as used in the present invention is a pharmaceutical preparation which is used as a dispersion in water upon administration. The pharmaceutical preparation to be dispersed before administration of the present invention may be a mixture of a active granules comprising a pharmaceutically active substance and a thickening agent at a given mixing ratio, being divided in portions. The pharmaceutical preparation can further comprise any of flavors, sweeteners and placebo granules. The pharmaceutical preparation to be dispersed before administration can also be divided powders or granules containing a active granules containing a pharmaceutically active substance and a thickening agent, respectively, which will be mixed upon administration. The "active granules" herein are granules containing a pharmaceutically active substance, and the "placebo granules" act as an extender and are used for improving handling upon administration.

The pharmaceutically active substance for use in the present invention can be in any form such as solid, powder, crystal, oil or solution. At least one selected from, for example, gastrointestinal drugs, re-vitalizer supplements, antipyretic analgesics antiphlogistic drugs, antipsychotic drugs, antianxiety drugs, antidepressant drugs, hypnosedative agents, antispastic drugs, antacids, antiussive and expectorants, agents for dental use, antihistamine drugs, heart stimulants, antiarrhythmics, diuretic agents, antihypertensive drugs, vasoconstrictors, coronary vasodilators, peripheral vasodilators, cholagogues, antibiotics, chemotherapeutic drugs, antidiabetic agents, drugs for treating osteoporosis and skeletal muscle relaxants may be used. The gastrointestinal drugs include digestants such as proton pump inhibitors, protection factor activator, H2 blockers, diastase, saccharated pepsin, scopolia extract, lipase AP or cinnamon oil; and drugs for controlling intestinal function such as berberine chloride, resistant lactic acid bacteria, and Bifidobacterium. The antacids include magnesium carbonate, sodium hydrogen carbonate, magnesium aluminometasilicate, precipitated calcium carbonate, synthetic hydrotalcite and magnesium oxide. The re-vitalizer supplements include vitamins including vitamin A, vitamin D, vitamin E such as d-α-tocopherol acetate, vitamin B1 such as dibenzoylthiamine or fursultiamine hydrochloride, vitamin B2 such as riboflavin butyrate, vitamin B6 such as pyridoxine hydrochloride, vitamin C such as ascorbic acid or sodium L-ascorbate, vitamin B12 such as hydroxocobalamin acetate; minerals such as calcium, magnesium or iron; proteins; amino acids; oligosaccharides; and crude drugs. Examples of the antipyretic analgesics antiphlogistic drugs are aspirin, acetaminophen, ibuprofen, ethenzamide, diphenhydramine hydrochloride, dl-chlorpheniramine maleate, dihydrocodeine phosphate, noscapine, methylephedrine hydrochloride, phenylpropanolamine hydrochloride, caffeine, serrapeptase, lysozyme chloride, tolfenamic acid, mefenamic acid, diclofenac sodium, flufenamic acid, salicylamide, aminopyrine, ketoprofen, indomethacin, bucolome and pentazocine. Examples of the antipsychotic drugs are amoxapine, chlorpromazine, sulpiride and reserpine. The antianxiety drugs include etizolam, bromazepam, chlordiazepoxide and diazepam. The antidepressant drugs include imipramine hydrochloride, maprotiline, clomipramine hydrochloride and amphetamine. The hypnosedative agents include flunitrazepam, triazolam, estazolam, nitrazepam, diazepam and phenobarbital sodium. The antiussive and expectorants include eprazinone hydrochloride, chloperastine hydrochloride, dextromethorphan hydrobromide, theophylline, potassium guaiacolsulfonate and guaifenesin. The agents for dental use include chloramphenicol, oxytetracycline, triamcinolone acetonide, chlorhexidine hydrochloride and lidocaine. The antihistamine drugs include azelastine hydrochloride, clemastine fumarate, diphenhydramine hydrochloride, promethazine, isothipendyl hydrochloride and dl-chlorpheniramine maleate. The heart stimulants include digitoxin and etilefrine hydrochloride. The antiarrhythmics include mexiletine hydrochloride, procainamide hydrochloride, disopyramide, propranolol hydrochloride and pindolol. The diuretic agents include trichlormethiazide, isosorbide and furosemide. Examples of the antihypertensive drugs are arotinolol hydrochloride, delapril hydrochloride, captopril, bisoprolol fumarate, hexamethonium bromide, hydralazine hydrochloride, labetalol hydrochloride and methyldopa. Examples of the vasoconstrictors are amezinium methylsulfate and phenylephrine hydrochloride. The coronary vasodilators include carbochromen hydrochloride, molsidomine and verapamil hydrochloride. The peripheral vasodilators include cinnarizine. The cholagogues include ursodeoxycholic acid, dehydrocholic acid and trepibutone. Examples of the antibiotics are cephem, penem and carbapenem antibiotics such as cefaclor, cephalexin, amoxicillin, faropenem sodium, pivmecillinam hydrochloride or cefotiam hydrochloride. The antidiabetic agents include glibenclamide, tolbutamide and voglibose. The drugs for treating osteoporosis include menatetrenone and ipriflavone.

The pharmaceutically active substance for use in the present invention is preferably a proton pump inhibitor.

Typically preferred pharmaceutically active substances in the present invention are benzimidazole compounds represented by the following formula 1, called as proton pump inhibitors. 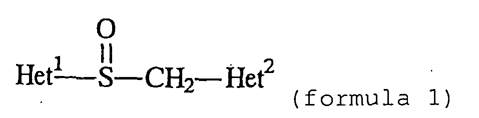 In the formula 1, Het¹ means 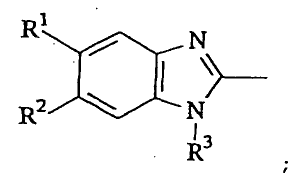 Het² means 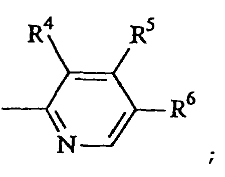 R¹ and R² are the same as or different from each other and are each selected from hydrogen, methoxy and difluoromethoxy; R³ is selected from hydrogen and sodium; and R⁴, R⁵ and R⁶ are the same as or different from each other and are each selected from hydrogen, methyl, methoxy, methoxypropoxy and trifluoroethoxy.

Preferred examples of the benzimidazole compounds in the present invention are rabeprazole, omeprazole and its optical isomer esomeprazole, pantoprazole and lansoprazole. Pharmaceutically acceptable salts thereof include sodium salts, potassium salts and magnesium salts. Structural formulae of these compounds are shown in the formula 2.

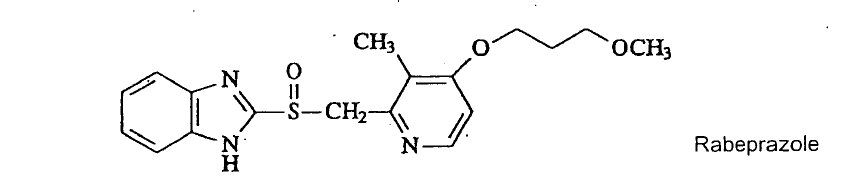

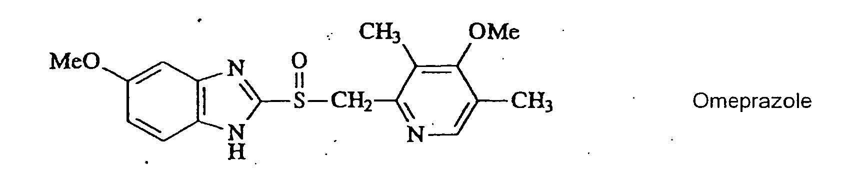

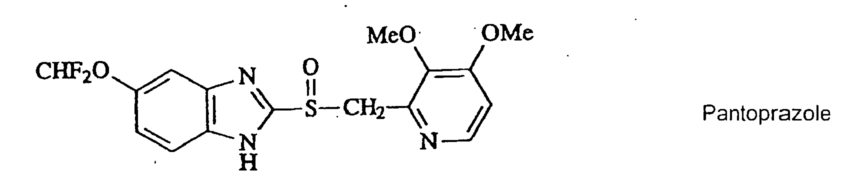

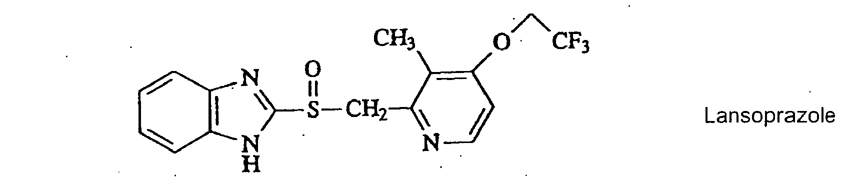

The "active granules" used in the present invention are granules containing a pharmaceutically active substance and containing one or more functional polymers. Examples of the active granules include, but are not limited to, coated granules comprising granules containing a pharmaceutically active substance coated with a functional polymer; and granules containing a functional polymer and a pharmaceutically active substance as a mixture (matrix granules).

The "functional polymer" refers to a polymer for controlling the release of a pharmaceutically active substance contained in the granules by dissolving or disintegrating under a desired condition of, for example, pH, organ, or time after administration and is not specifically limited. A layer coated with the functional polymer is referred to as a "functional coat". Examples of the functional polymers are gastric polymers, enteric polymers, and sustained release polymers. Any gastric polymer can be arbitrarily selected herein, and such gastric polymers include, but are not specifically limited to, hydroxypropylmethyl cellulose, hydroxypropyl cellulose, polyvinyl acetal diethylaminoacetate, and aminoalkyl methacrylate copolymers. Any enteric polymer can be arbitrarily selected in the present invention, and such enteric polymers include, but are not specifically limited to, hydroxypropylmethyl cellulose phthalate, hydroxypropylmethyl cellulose acetate succinate, carboxymethylethyl cellulose, methacrylic acid copolymer L and methacrylic acid copolymer LD. Any sustained release polymer can be arbitrarily selected in the present invention, and such sustained release polymers include, but are not specifically limited to, hydroxypropylmethyl cellulose acetate succinate, methacrylic acid copolymer S, aminoalkyl methacrylate copolymer RS and ethyl cellulose. Each of these functional polymers can be used alone or in combination.

Coated granules, if used as the active granules in the present invention, can be produced by coating a regular granulated substance with a functional polymer, or by coating a core substance (seed) with a functional polymer. The coated granules can have plural coats of functional polymers. In this case, a coat of a functional polymer can be applied on another coat of another functional polymer with or without the interposition of an intermediate coat.

When the coated granules are produced by granulation, they can be produced mixing, granulating, and sizing a pharmaceutically active substance with additives such as a filler and a stabilizer, and coating the granules with a functional polymer, or by granulating the other components than the pharmaceutically active substance and coating the resulting granules with the pharmaceutically active substance.

When the coated granules are produced by coating a core substance (seed), the core substance is a substance serving as a core or seed to which surface a layer of a pharmaceutically active substance, additives and other components is applied typically by adsorption to form granules. The material for the seed is not specifically limited, and any of commercially available granules or spherical granules prepared by mixing, granulating and sizing various additives will do. The seed can comprise any component but preferably comprises a substance having a low reactivity with the pharmaceutically active substance, such as mannitol or crospovidone. The core substance is generally spherical, and the average particle diameter thereof can be about 80 µm to about 800 µm and is preferably about 100 µm to about 700 µm, and more preferably about 100 µm to about 500 µm. Preferred examples of the core substance are NONPAREIL 103 (Freund Corporation), NONPAREIL 108 (Freund Corporation) and Celphere (Asahi Chemical Industry Co., Ltd.).

The "matrix granules" as the active granules refer to granules comprising a mixture of a functional polymer and a pharmaceutically active substance. These granules can be prepared by mixing a functional polymer and a pharmaceutically active substance, where necessary further with additives such as a filler and a stabilizer, and granulating and sizing the mixture, or molding the mixture into granules typically under the application of pressure. The granulation process includes, but is not specifically limited to, wet granulation, dry granulation, extrusion granulation, and melting granulation.

The active granules can have such a particle diameter as to pass through an NG tube having an inner diameter of 5 mm. The particle diameter is, for example, about 50 µm to about 4900 µm, preferably about 100 µm to about 800 µm, more preferably about 200 µm to about 500 µm, and further preferably about 200 µm to about 400 µm.

For coating layers of the pharmaceutically active substance and the functional coat, any of water-soluble polymers, water-insoluble polymers and water-dispersible polymers can be used. Preferred examples of the water-soluble polymers include, but are not limited to, hydroxypropyl cellulose (HPC), hydroxypropylmethyl cellulose (HPMC), methyl cellulose (MC) and polyvinyl alcohol (PVA), of which HPC is preferred. As HPC, HPC-L (Nippon Soda Co., Ltd.), for example, is preferably used. Crospovidone can also be used as the water-soluble polymer in the present invention. Examples of the crospovidone are CL and CLM (BASF AG); INF-10, XL-10, and XL (ISP Japan), of which Polyplasdone INF-10 (ISP Japan) having a smaller particle diameter or Crospovidone XL having a less content of peroxides is preferred. Crospovidone XL is advantageously used for an oxidation-labile pharmaceutically active substance.

The water-insoluble polymers for use in the present invention include, but are not limited to, ethyl cellulose, butyl cellulose, cellulose acetate, polyvinyl acetate, cellulose propionate, polyvinyl butyrate, Eudragit RS (Rohm Pharma GmbH: a copolymer of ethyl acrylate, methyl methacrylate and trimethylammonium ethyl methacrylate chloride).

The "matrix granules" as the active granules in the present invention refer to granules comprising a functional polymer and a pharmaceutically active substance as a mixture. These granules can be prepared by mixing a functional polymer and a pharmaceutically active substance, where necessary further with additives such as an excipient and a stabilizer, and granulating and sizing the mixture, or by molding into granules typically under the application of pressure. The granulation process includes, but is not specifically limited to, wet granulation, dry granulation, extrusion granulation and melting granulation.

The thickening agent for use in the present invention can be arbitrarily selected and includes, but is not limited to, methyl cellulose, propylene glycol alginate (available in the trade name of Kimiloid from Kimica Corporation), xanthan gum, purified gelatin, HPC, HPMC, PVA, polyvinylpyrrolidone (PVP), sodium polycarboxymethyl cellulose (CMC-Na), macrogol and povidone. HPC-M and HPC-L are preferably used as HPC. Metolose SM (Shin-Etsu Chemical Co., Ltd.), Cellogen (Daiichi Kogyo Seiyaku co., Ltd.) and Metolose 65SH (Shin-Etsu Chemical Co., Ltd.) can be used as the methyl cellulose, CMC-Na and HPMC, respectively. Of these thickening agents, propylene glycol alginate and methyl cellulose are preferably used. The sweetener can be selected arbitrarily, for example, from among aspartame, stevia, hydrogenated malt syrup, xylitol, dipotassium glycyrrhizinate, disodium glycyrrhizinate, saccharin, saccharin sodium, purified sucrose, D-sorbitol, lactose, sucrose and maltitol.

The pharmaceutical preparation can further comprise placebo granules as an extender for the active granules and for improving the handleability upon administration. The placebo granules are not specifically limited but are preferably granules having a size and a density similar to those of the active granules. The formulation of the placebo granules is not limited. The placebo granules may further comprise a thickening agent. They can be produced, for example, by blending and pulverizing mannitol, crospovidone, citric acid and light anhydrous silicic acid, granulating the mixture with purified water, drying and sizing the granules.

Any flavor, such as strawberry flavor, orange extract, vanilla flavor or mint flavor, can be selectively used in the present invention.

The pharmaceutical preparation to be dispersed before administration of the present invention can be prepared by 1) mixing active granules and a thickening agent or 2) mixing active granules, placebo granules and a thickening agent. The amount of the thickening agent is preferably about 0.5 to about 50 parts by weight, and more preferably about 1 to about 10 parts by weight per 1 part by weight of the granules. The amount of the placebo granules, if used, is preferably 1 to 400 parts by weight, and more preferably 3 to 300 parts by weight per 1 part by weight of the active granules. The amount of the pharmaceutical preparation to be dispersed before administration per pack is generally 0.1 to 20 g and preferably 0.3 to 10 g. The amount of water for dispersion is generally about 1 to about 100 ml and preferably about 2 to about 50 ml. The kinematic viscosity of the resulting dispersion is generally about 10 to about 1500 mPa.s, preferably about 15 to about 1000 mPa.s, and more preferably about 50 to about 600 mPa.s.

The water-insoluble polymers for use in the present invention include, but are not limited to, ethyl cellulose, butyl cellulose, cellulose acetate, polyvinyl acetate, cellulose propionate, polyvinyl butyrate and Eudragit RS (Rohm Pharma GmbH: a copolymer of ethyl acrylate, methyl methacrylate and trimethylammonium ethyl methacrylate chloride).

Active granules containing a proton pump inhibitor as the pharmaceutically active substance can be produced, for example, in the following manner. The production method, however, is not limited thereto.

The active granules are prepared by (1) coating a core substance with crospovidone to form adsorbed granules; (2) coating the adsorbed granules with sodium hydroxide and a benzimidazole compound to form active adsorbed granules; (3) coating the active adsorbed granules with ethyl cellulose, HPC-L and magnesium stearate to form undercoated granules; and (4) coating the undercoated granules with hydroxypropylmethyl cellulose, monoglyceride, talc and titanium oxide to form enteric-coated granules.

In the step (1) of forming adsorbed granules, a water-soluble polymer such as HPC or HPMC is dissolved in ethanol or water, crospovidone is dispersed in the solution to form a dispersion, and the dispersion is applied to a core substance. Coating can be carried out, for example, by using CF, a fluidized-bed coating granulator or a Wurster fluidized-bed coating granulator. The adsorbed granules bearing a crospovidone layer have a particle diameter of generally about 50 to about 900 µm and preferably about 100 to about 800 µm. The amount of crospovidone is not limited and is generally 0.1 to 100 parts by weight, preferably 1 to 50 parts by weight and more preferably 3 to 20 parts by weight per 1 part by weight of the benzimidazole compound. When crospovidone granules are used as the core substance as mentioned above, this step can be omitted.

In the step (2) of forming active adsorbed granules from the adsorbed granules, sodium hydroxide can be applied before or simultaneously with the coating of the pharmaceutically active substance. For example, a solution of sodium hydroxide in ethanol or water is applied to the adsorbed granules, and a coating solution of the pharmaceutically active substance (the benzimidazole compound) in ethanol or water is applied thereto. The coating procedure is as in the production of the adsorbed granules. The amount of sodium hydroxide can be adjusted according to the amount of the benzimidazole compound and is generally 0.1 to 5 parts by weight, preferably 0.2 to 4 parts by weight, and more preferably 0.2 to 3 parts by weight per 1 part by weight of the benzimidazole compound.

The undercoating in the step (3) is coating for preventing direct contact of an acid-labile pharmaceutically active substance with an enteric basis, an acidic substance, used in the subsequent enteric coating. The undercoat can be any of water-soluble coats, water-insoluble coats and water-dispersible coats. The undercoated granules are produced, for example, by dissolving ethyl cellulose and HPC-L in ethanol or water, adding magnesium stearate to the solution to form a dispersion as a coating composition, and applying the coating composition to the active adsorbed granules.

The enteric coat formed in the step (4) is an enteric coat that is insoluble at pH in the stomach but soluble at pH in the intestine. The enteric basis as a main component of the enteric coat includes, but is not limited to, hydroxypropylmethyl cellulose phthalate (HP-55S), cellulose acetate phthalate (CAP), methacrylic acid copolymer type A (Eudragit L) and methacrylic acid copolymer type C (L-55). The enteric coat granules are produced, for example, by dissolving HP-55S and monoglyceride in ethanol or water, dispersing talc and titanium oxide in the solution to form a coating composition, and applying the coating composition to the active adsorbed granules.

The active granules and the placebo granules in the present invention may further comprise any of excipients, binders, coating agents, colorants, flavors and other components generally used in manufacturing process, in addition to the above-mentioned components.

Active granules obtained in Examples 1 to 6 are pharmaceutical compositions having a layer (1) containing crospovidone, and a layer (2) containing sodium hydroxide and a benzimidazole compound or a pharmacologically acceptable salt thereof and being arranged adjacent to the layer (1). The present inventors have surprisingly found that, when a crospovidone layer and a layer containing sodium hydroxide and a benzimidazole compound are separately coated, sodium hydroxide is adsorbed by the crospovidone layer and the benzimidazole compound can be further efficiently coated.

The pharmaceutical preparation to be dispersed before administration according to the present invention becomes highly flowable when dispersed in water before administration and can be easily taken even by infants and children. According to the present invention, a pharmaceutical preparation to be dispersed before administration having an adequate viscosity and suitable flowability can be obtained regardless of the type of the pharmaceutically active substance contained in the active granules.

### Brief Description of Drawings

Fig. 1 is a graph showing the dissolution profile of active granules obtained in Example 1.
Fig. 2 is a graph showing the dissolution profiles of active granules obtained in Examples 2 and 3.

### Examples

The present invention will be illustrated in further detail with reference to Examples below which by no means limit the scope of the present invention.

### Example 1

A Wurster fluidized-bed drying granulator (MP-01, Powrex Corporation) was used as a coating machine.

### Adsorbed granules

A core substance was coated with HPC-L dissolved and crospovidone dispersed in ethanol at an intake gas temperature of 55°C. The coated granules were dried at 50°C in a rack dryer.

### Active adsorbed granules

The above-mentioned adsorbed granules were coated with a solution of sodium hydroxide in ethanol and then coated with a benzimidazole compound and HPC-L dissolved in ethanol at an intake gas temperature of 55°C. The coated granules were dried at 50°C in a rack dryer.

### Undercoated granules

Ethyl cellulose and HPC-L were dissolved in ethanol, and magnesium stearate was added thereto and dispersed. The above-mentioned active adsorbed granules were coated with the resulting solution at an intake gas temperature of 55°C. The coated granules were dried at 45°C in a rack dryer to give undercoated granules.

### Enteric-coated granules

HP-55S and Myvacet were dissolved in ethanol, and talc and titanium oxide were dispersed therein.. The above-mentioned undercoated granules were coated with the resulting solution at an intake gas temperature of 55°C to give enteric-coated granules.

### Examples 2 to 6: Active granules

A series of pharmaceutical compositions as active granules was produced by the same procedure as Example 1. The formulations of the produced active granules are shown in Table 1.

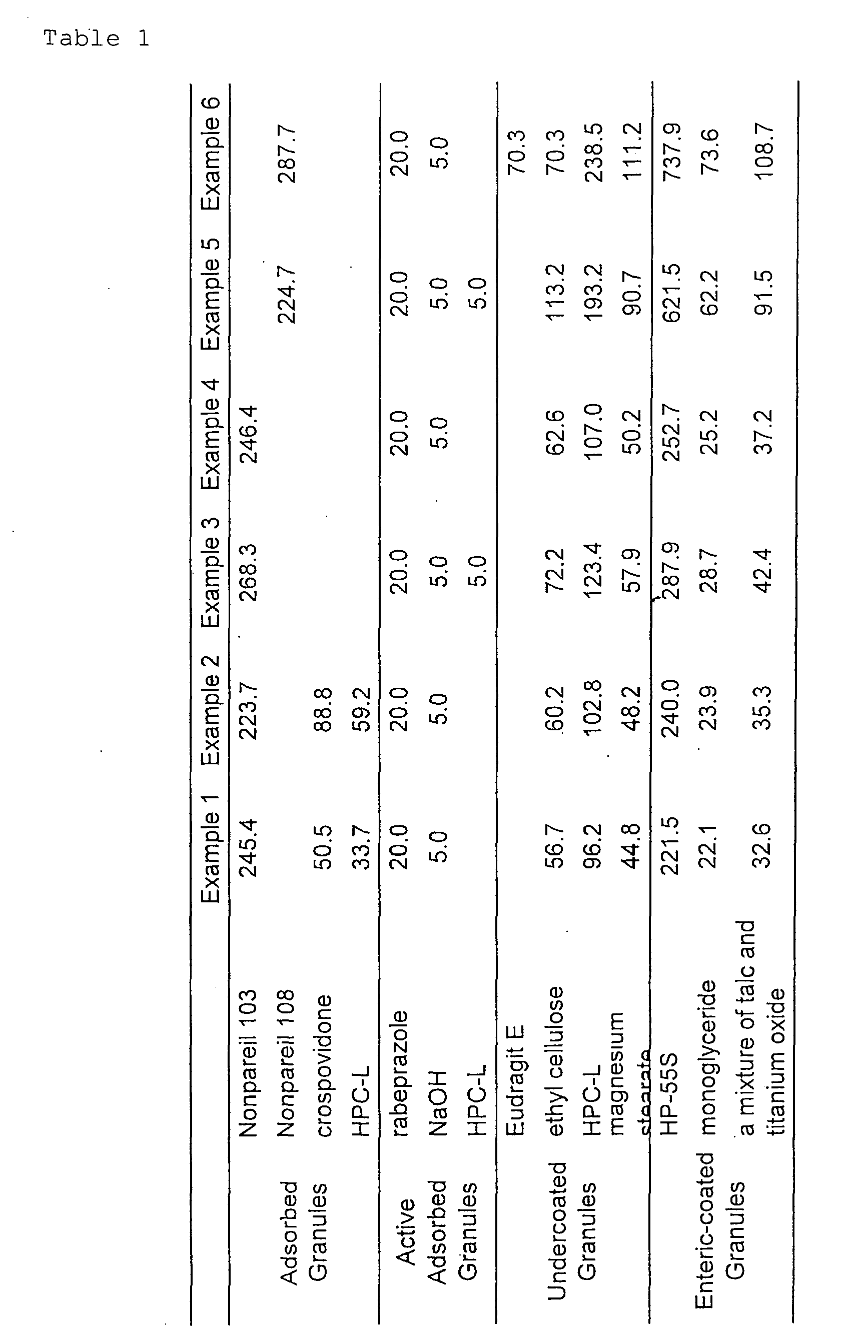

### Example 7: Placebo granules

Placebo granules were prepared by mixing and pulverizing mannitol, crospovidone, citric acid and light anhydrous silicic acid, granulating the mixture with purified water, drying and sizing the granules. A thickening agent, aspartame and strawberry flavor were added thereto to give a mixture containing the placebo granules. The formulation thereof is shown in Table 2.

**Table 2**

| | |
|---|---|
| D-Mannitol | 2190 |
| Crospovidone XL | 300 |
| Light anhydrous silicic acid | 100 |
| Citric acid | 10 |
| | |
| Methyl cellulose | 300 |
| Aspartame | 10 |
| Strawberry flavor | 3 |

### Example 8: Pharmaceutical preparation to be dispersed before administration

A pharmaceutical preparation to be dispersed before administration was prepared by mixing the active granules obtained in Example 1 and the mixture containing placebo granules obtained in Example 7 in proportions by weight of 1:3.4.

### Examples 9 and 10

Placebo granules were prepared by mixing and pulverizing mannitol, citric acid and PEG, granulating the mixture with ethanol, and drying and sizing the granules. Methyl cellulose or propylene glycol alginate, a sweetener and a flavor were added thereto to give a mixture containing placebo granules. The placebo granules obtained in these Examples 9 and 10 contain a thickening agent (methyl cellulose or propylene glycol alginate) in the placebo granules, in contrast to the placebo granules obtained in Example 7. The formulations are shown in Table 3.

**Table 3**

| | Example 9 | Example 10 |
|---|---|---|
| D-Mannitol | 1401.5 | 1341.5 |
| Citric acid | 7.0 | 7.0 |
| PEG8000 | 130.0 | 130.0 |
| | | |
| Propylene glycol alginate | 160.0 | - |
| Methyl cellulose | - | 220.0 |
| L-HPC | 200.0 | 200.0 |
| Aspartame | 60.0 | 60.0 |
| Strawberry flavor | 40.0 | 40.0 |

### Examples 11 to 17: Other placebo granules

Mixtures containing placebo granules were prepared (Examples 11 to 17) in substantially the same way as in Examples 9 and 10. The formulations are shown in Table 4.

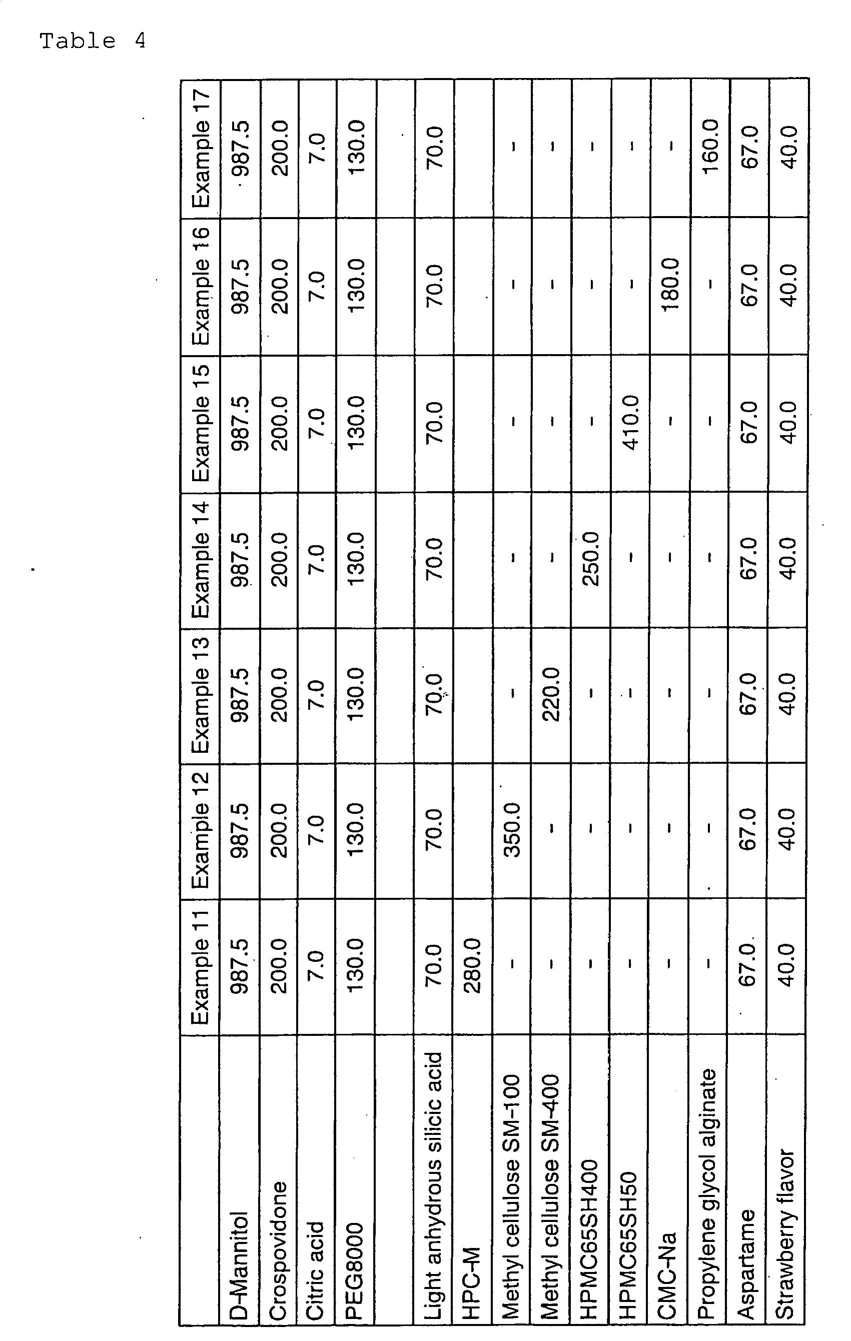

### Experimental Example

The active granules and the pharmaceutical preparations to be dispersed before administration produced in the above Examples were subjected to a dissolution test, a stability test and a physical property test.

### Dissolution test

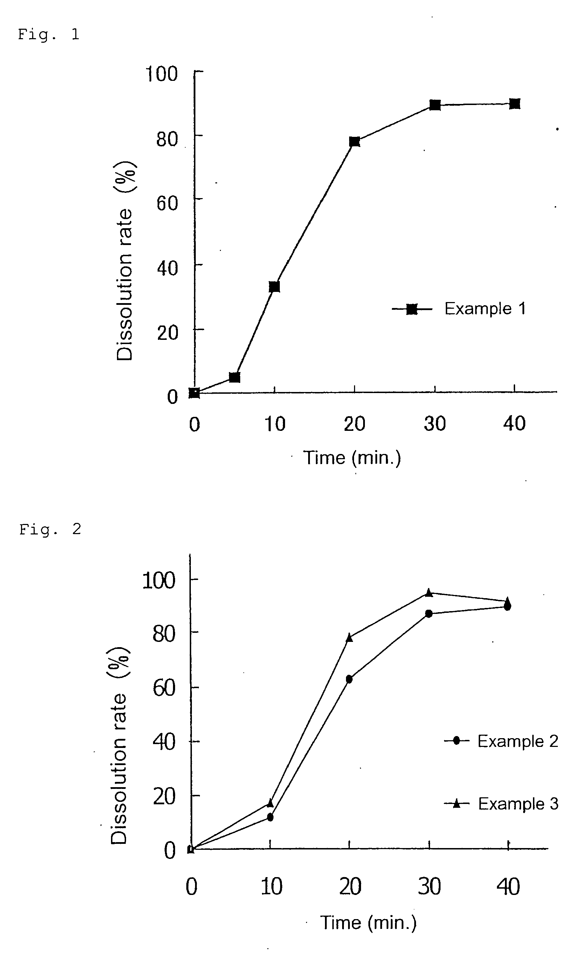

The pharmaceutical preparations obtained in Examples 1 to 3 were subjected to a dissolution test. These active granules are enteric-coated granules, and the dissolution test was conducted by paddle method (100 revolutions per minute) using the second liquid having a pH of about 6.8 as specified in the disintegration test of Japanese Pharmacopoeia as a dissolution test liquid. The results of the dissolution test are shown in Figs. 1 and 2. Figs. 1 and 2 demonstrate that the active granules using the pharmaceutical compositions of the present invention have satisfactory dissolution properties.

### Stability test

The stability of the pharmaceutically active substance of each pharmaceutical preparation was evaluated based on the amount of impurities formed by decomposition of the pharmaceutically active substance when the active granule was stored under set conditions. The active granules obtained in Example 1 were stored at 5°C and 25°C at relative humidity of 60%, respectively, for three months, and the total amount of impurities of the granules was determined. In contrast, initial total amounts of impurities of the active granules obtained in Examples 2 and 3 were determined. The results are shown in Table 5.

**Table 5**

| Example No. | Example 1 | | | Example 2 | Example 3 |
|---|---|---|---|---|---|
| storage conditions | initial | 5°C | 25°C 60% RH | initial | initial |
| storage period | - | 3M | 3M | - | - |
| the total amounts of impurities | 0.85 | 0.84 | 1.09 | 0.95 | 0.81 |

Table 5 demonstrates that the active granules obtained in Example 1 are a stable pharmaceutical preparation which does not substantially undergo decomposition of the pharmaceutically active substance even after a long-term storage. Further, the active granules obtained in Examples 2 and 3 have very small amounts of impurities immediately after production.

### Physical property test

The physical properties as a powder (bulk density, tapped density, Carr's compressibility index and flowability) and the viscosity as a dispersion in water of the pharmaceutical preparation to be dispersed before administration obtained in Example 8 were determined. The results are shown in Table 6.

**Table 6**

| | |
|---|---|
| Bulk density | 0.54 |
| Tapped density | 0.67 |
| Carr's compressibility index | 24 |
| Flowability | good |
| the minimum required amount of water to swallow | five spoonfuls |
| Viscosity (Pa·s) | 0.2652 |

It is evident in Table 6 that a good dispersibility and an adequate viscosity were achieved by adding five spoonfuls of water (about 20 ml). The pharmaceutical preparation of the present invention had such flowability as to be administered to an infant using an NG tube (a tube for administering a drug through the nasal cavity) having an inner diameter of 5 mm, in which the active granules were adequately dispersed without aggregation. The granules showed no aggregation in water. The pharmaceutical preparation according to the present invention was a good pharmaceutical preparation to be dispersed before administration.

### Viscosity determination

Two grams (2 g) of each of the mixtures containing placebo granules obtained in Examples 11, 12, 13 and 17 was dissolved in 10 ml of purified water, and the kinematic viscosity of the solution was determined with a rheometer. The results are shown in Table 7.

**Table 7**

| | | | | |
|---|---|---|---|---|
| Unit: mPa·s | | | | |

| Examples | Example 11 | Example 12 | Example 13 | Example 17 |
|---|---|---|---|---|
| Viscosity | 754 | 402 | 523 | 359 |

### NG tube passability test

In 10 ml of water were dispersed 2 g of the placebo granules obtained in Example 9 and 300 mg of active granules having an average particle diameter of about 470 µm, and then the dispersion was sucked into a disposable syringe. An NG tube (5 Fr) having an inner diameter of 1 mm was attached to the syringe. A pressure was then applied to the syringe, and whether or not the pharmaceutical preparation to be dispersed before administration of the present invention flowed out from the NG tube was determined. Consequently, the pharmaceutical preparation to be dispersed before administration of the present invention flowed out from the NG tube, indicating good flowability.

## Claims

1. A pharmaceutical preparation to be dispersed before administration, comprising active granules comprising a pharmaceutically active substance and having an average particle diameter of 2 mm or less; and a thickening agent, wherein the pharmaceutical preparation is capable of being administered through an NG tube by dispersing in water before administration.

2. The pharmaceutical preparation to be dispersed before administration according to claim 1, wherein the active granules further comprise a functional polymer.

3. The pharmaceutical preparation to be dispersed before administration according to claim 2, wherein the functional polymer is at least one selected from the group consisting of gastric polymers, enteric polymers and sustained release polymers.

4. The pharmaceutical preparation to be dispersed before administration according to any one of claims 1 to 3, wherein the thickening agent is at least one selected from the group consisting of propylene glycol alginate, methyl cellulose, hydroxypropylmethyl cellulose, polyvinylpyrrolidone, sodium polycarboxymethyl cellulose and hydroxypropyl cellulose.

5. The pharmaceutical preparation to be dispersed before administration according to any one of claims 1 to 4, further comprising placebo granules containing no pharmaceutically active substance.

6. The pharmaceutical preparation to be dispersed before administration according to any one of claims 1 to 5, wherein the pharmaceutical preparation has a viscosity of 10 to 1500 mPa·s when dispersed in water before administration.

7. The pharmaceutical preparation to be dispersed before administration according to any one of claims 1 to 6, wherein the pharmaceutically active substance is a proton pump inhibitor.

8. The pharmaceutical preparation to be dispersed before administration according to claim 7, wherein the proton pump inhibitor is at least one selected from the group consisting of rabeprazole, omeprazole, esomeprazole, lansoprazole and pantoprazole.
